# EUROPEAN PATENT APPLICATION

(11) **EP 1 997 907 A1**
(43) Date of publication of application: **03.12.2008**
(21) Application number: 07109444.5
(22) Date of filing: 01.06.2007
(51) Int. Cl.: C12Q 1/68

(54) **Bifidobacteria**

(71) Applicant: Friesland Brands B.V., 7943 PE Meppel (NL)
(72) Inventor: Nauta, Arjen, 6721 CG Bennekom (NL); van den Heuvel, Elisabeth Gertruda Hendrika Maria, 3572 GS Utrecht (NL); Veurink, Janine Henriët, 7681 SK Vroomshoop (NL); Geurts, Johannes Marie Wilhelmus, 7559 NN Hengelo (NL)
(74) Representative: van Loon, C.J.J.

(57) **Abstract**

The invention provides means and methods for detecting, amplifying, quantifying and/or isolating nucleic acid of at least one member of *Bifidobacterium.*

## Description

The invention relates to the fields of biology and microbiology.

*Bifidobacterium* is a genus of anaerobic bacteria which are abundantly present in the gastrointestinal tract. The term Bifidobacteria is a generic term referring to a group of organisms belonging to the genus *Bifidobacterium.* This group is classified into at least 30 (sub-)species, of which several inhabit the intestinal lumen of mammals (including humans). Bifidobacteria have health-promoting properties since they improve food uptake and they are associated with resistance to gastroenterological infections and resistance to tumors such as colon cancer. They are also associated with a lower incidence of allergies.

The presence of Bifidobacteria in the gastrointestinal tract is important for the well-being of individuals. A decrease of the amount of Bifidobacteria, for instance resulting from immune depression, disease, stress or the use of antibiotics, other drugs or excess alcohol, adversely influences an individual's well-being. A diminished amount of Bifidobacteria has an influence on the general health status and often coincides with an increase in susceptibility to gastroenterological infections and chronic diseases like colon cancer. The amount of Bifidobacteria also alters with increasing age. Lower numbers of Bifidobacteria are present in the bacterial flora in human individuals over 60 years of age, compared to younger healthy adults.

A sufficiently high amount of Bifidobacteria is for instance obtained and/or maintained by the uptake of probiotics and/or prebiotics. Probiotics and prebiotics are useful for improving and/or restoring a normal healthy amount of Bifidobacteria in an individual's digestive tract. Probiotics are live micro-organisms, such as *Bifidobacterium,* that are capable of improving the balance of the intestinal microflora. Probiotic bacterial cultures assist the body's naturally occurring flora within the digestive tract to reestablish themselves. Prebiotics are food ingredients that beneficially affect the host by selectively stimulating the growth and/or the activity of a limited number of bacteria in the colon.

The presence and/or amount of Bifidobacteria in a sample, such as a biological sample or a food sample, is often determined using a *Bifidobacterium-specific* probe. Nucleic acid present in a sample is often amplified using *Bifidobacterium*-specific primers, after which the presence of *Bifidobacterium-*specific nucleic acid is detected. During such detection methods, false positive results and false negative results are preferably avoided. False positive results are obtained if nucleic acid other than *Bifidobacterium* nucleic acid is detected. False negative results are obtained if nucleic acid of one or more strains of *Bifidobacterium* present in a sample is not detected. Currently used nucleic acid detection methods are often not specific enough (yielding false-positive results) or too specific, so that nucleic acid of one or more *Bifidobacterium* strains are not detected (yielding false-negative results).

It is an object of the present invention to provide means and methods for detecting, amplifying, identifying and/or isolating *Bifidobacterium* nucleic acid.

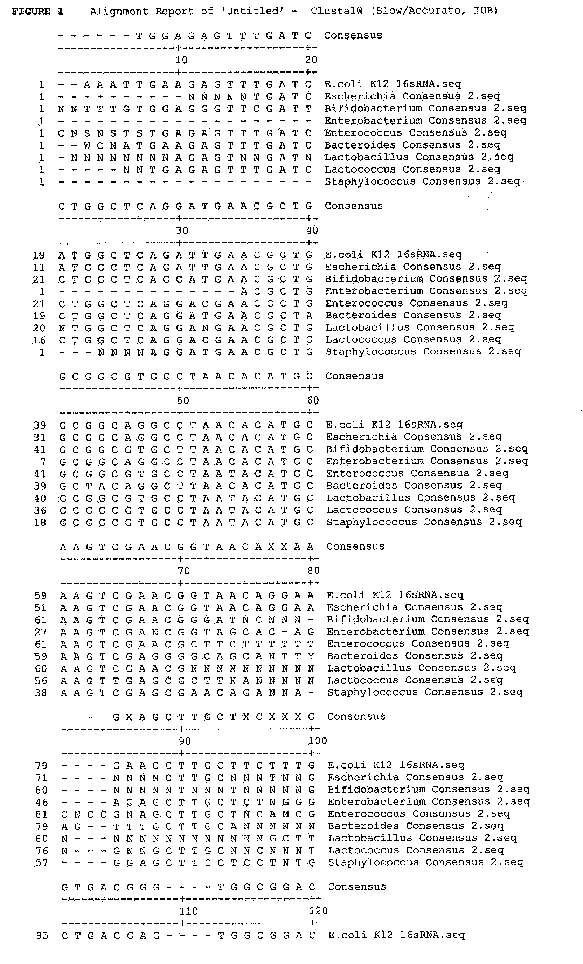

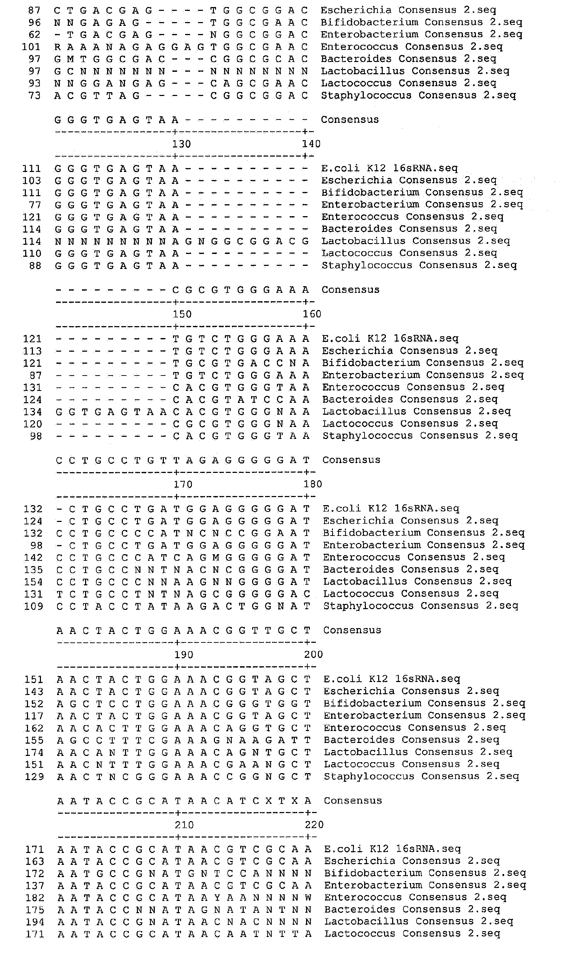

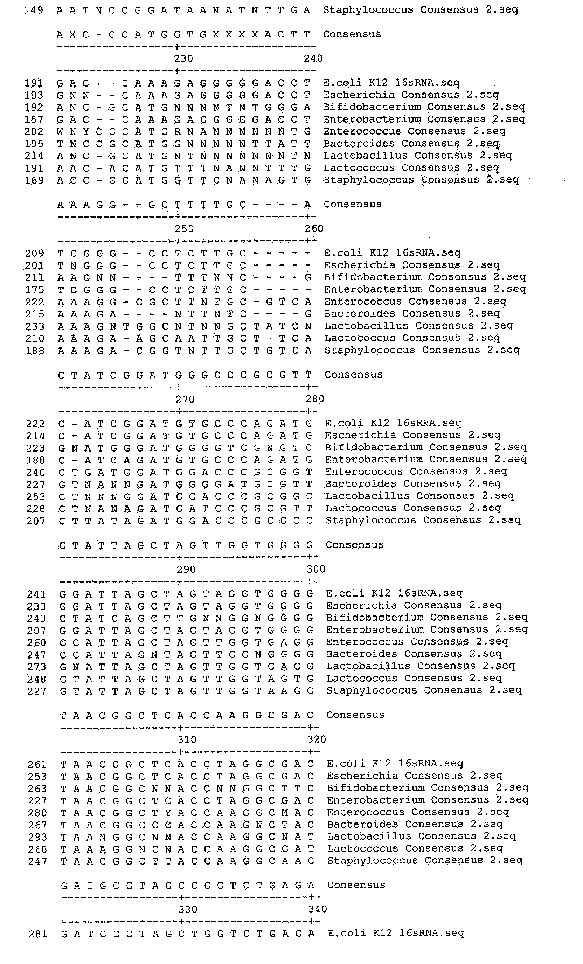

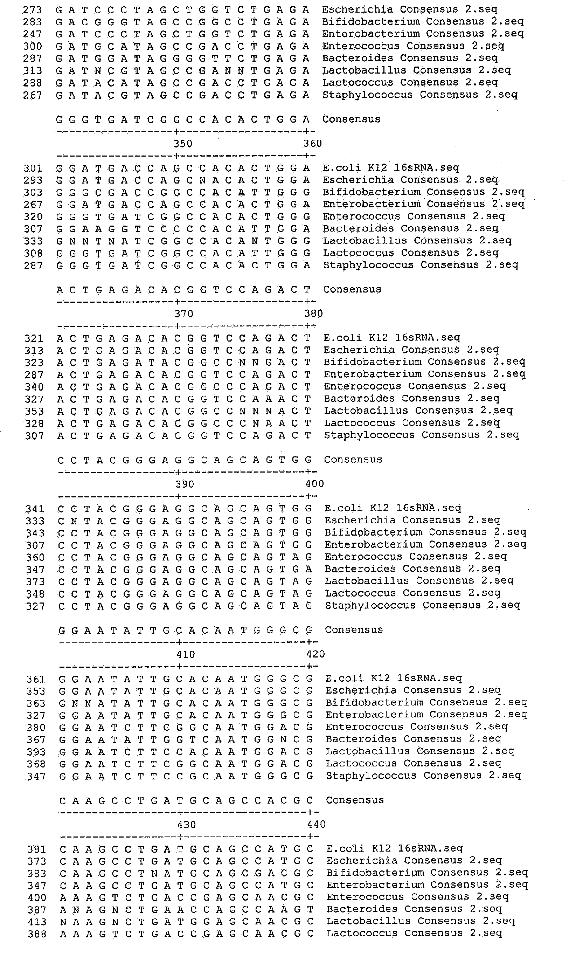

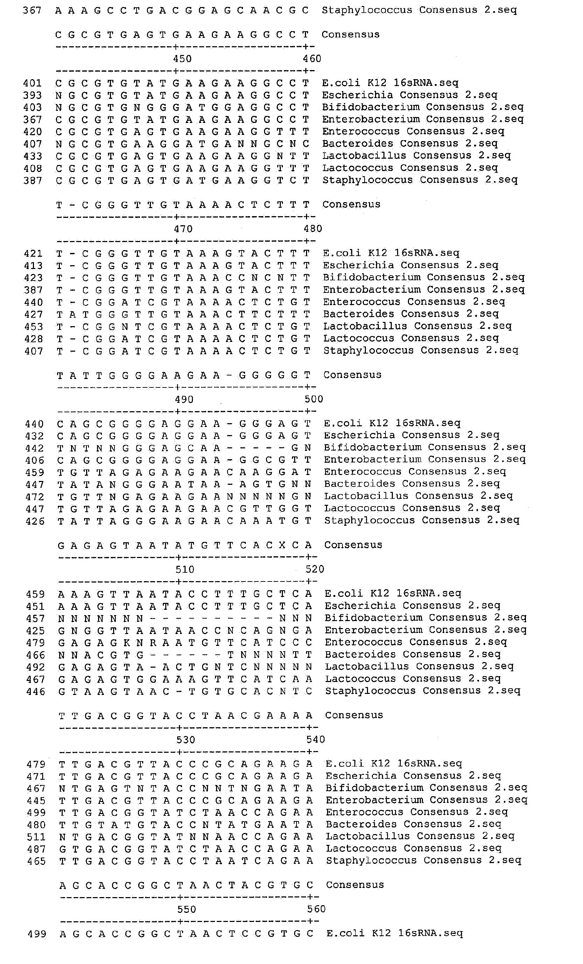

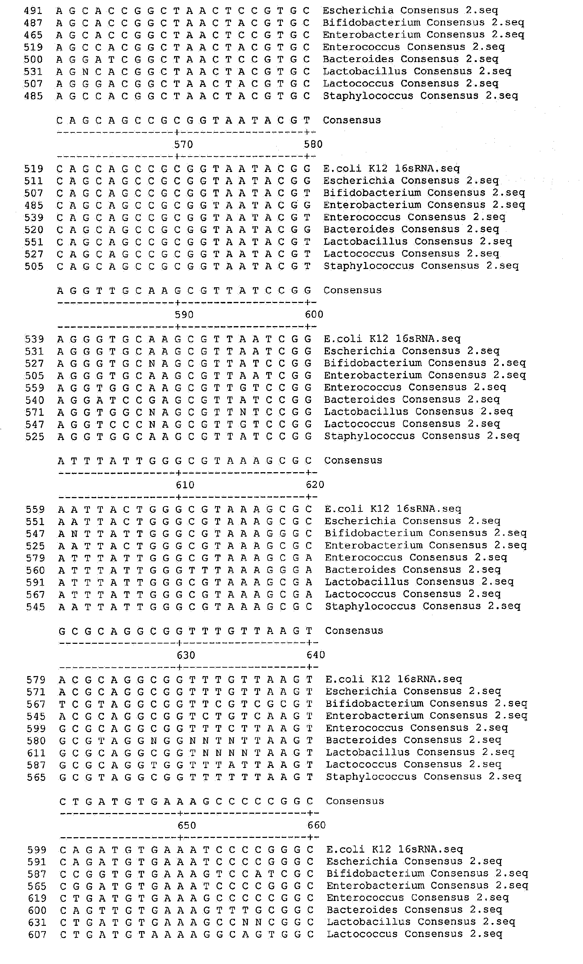

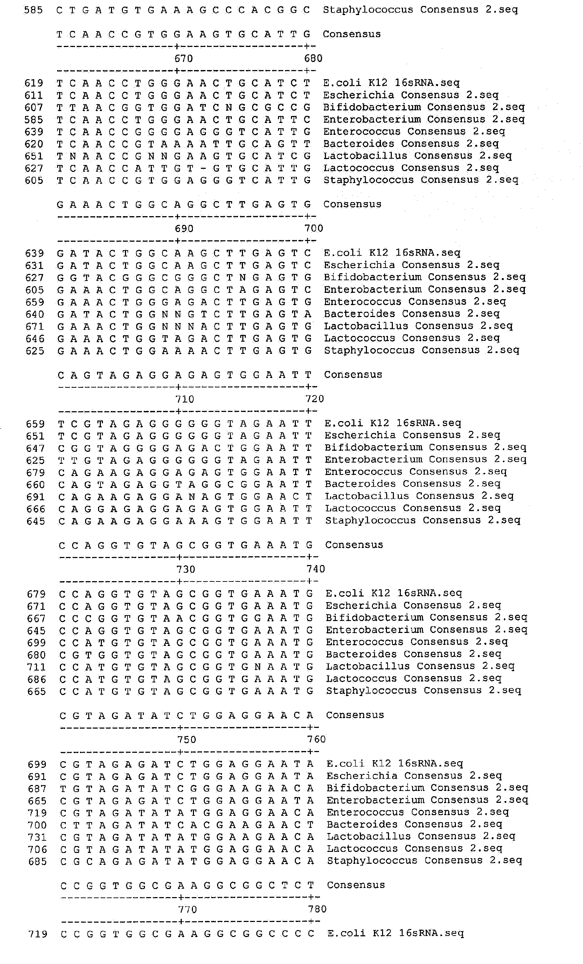

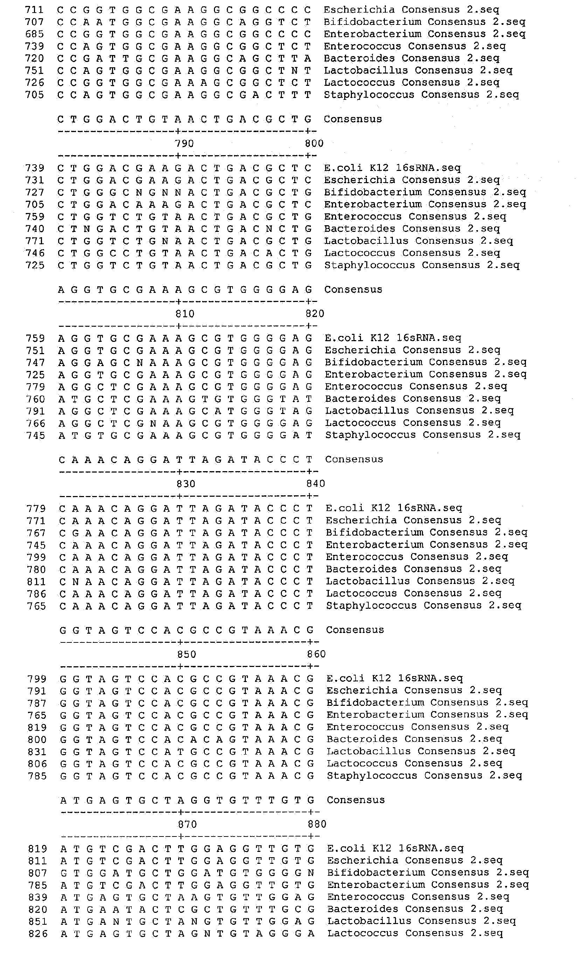

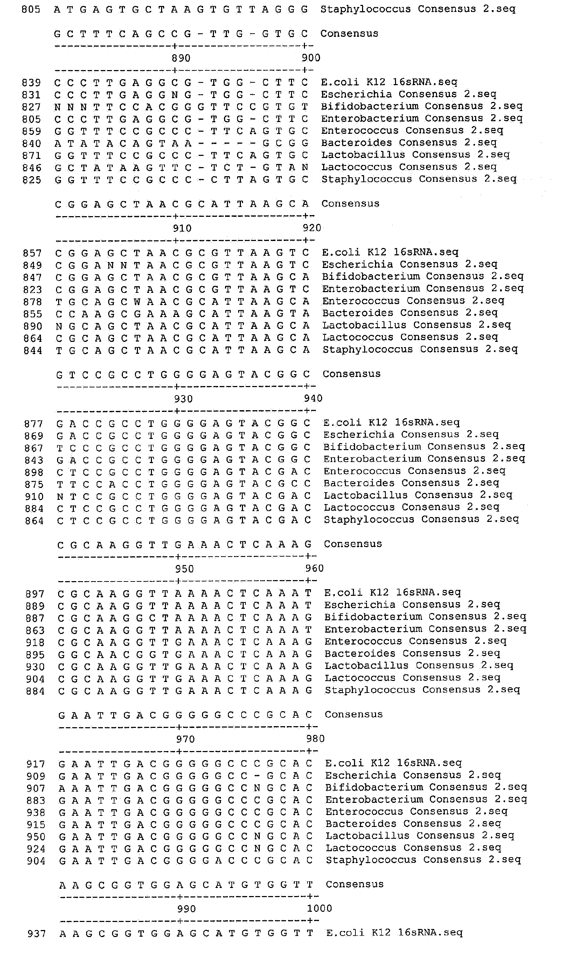

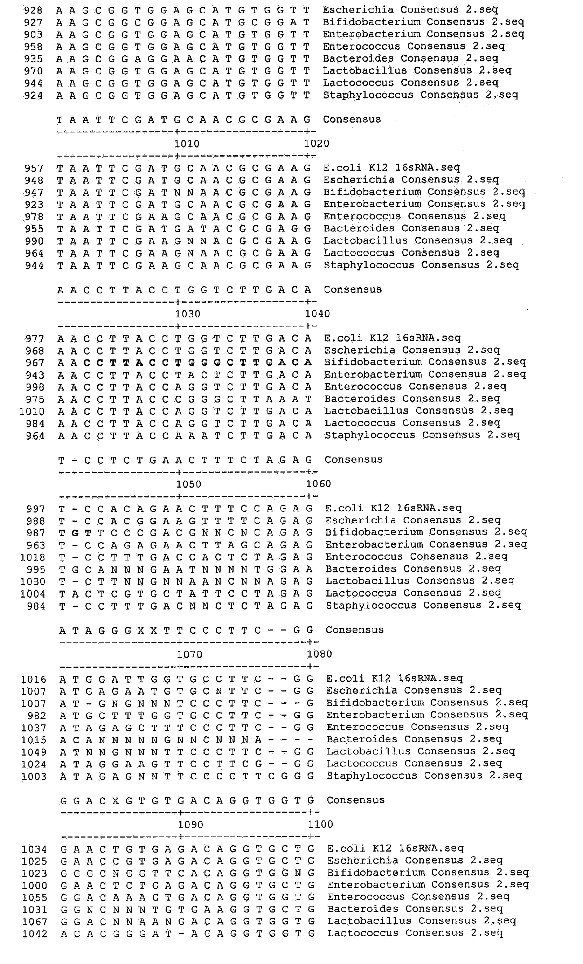

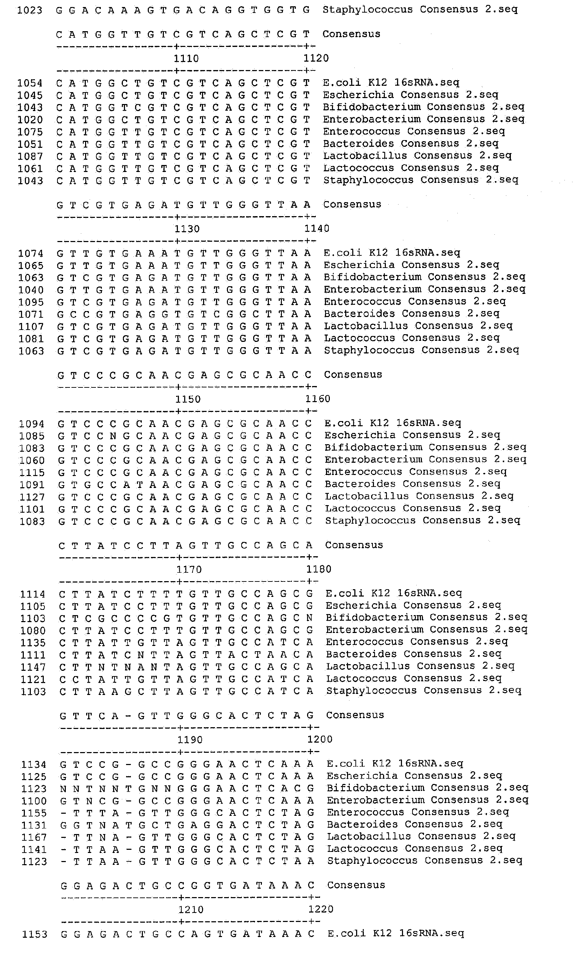

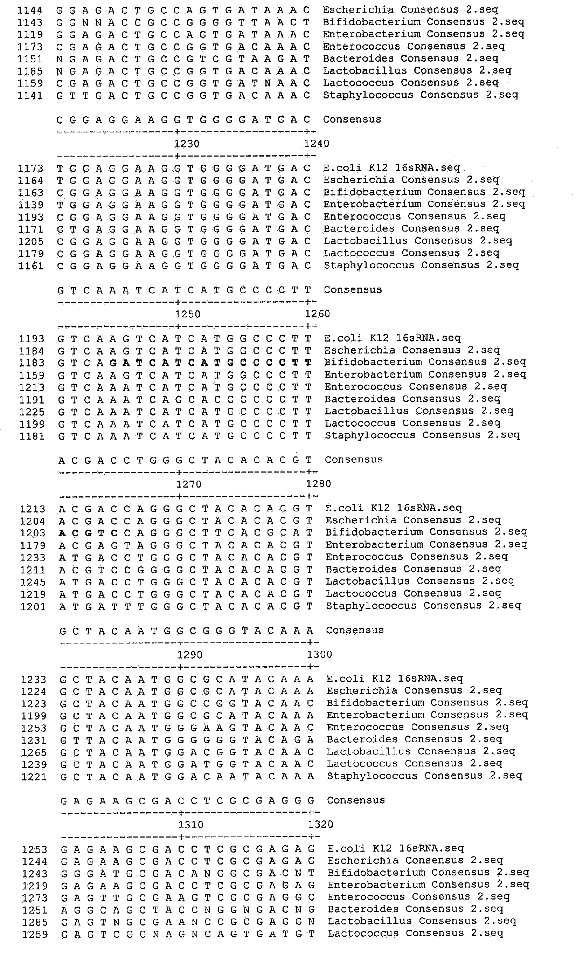

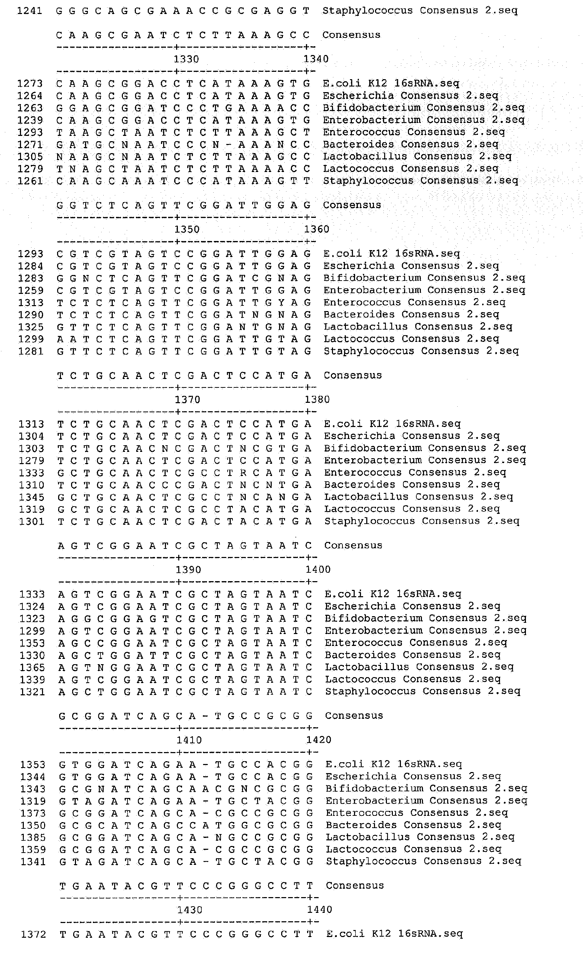

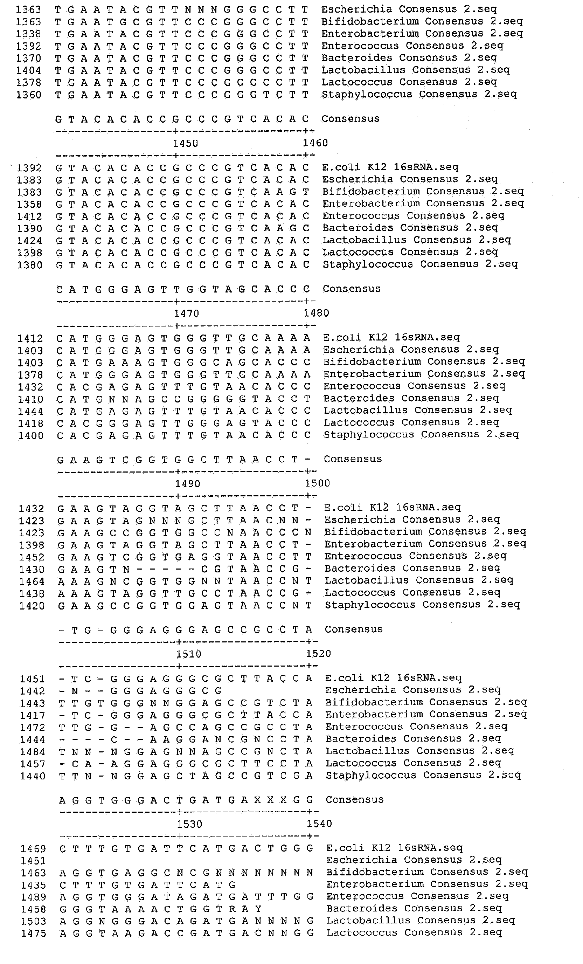

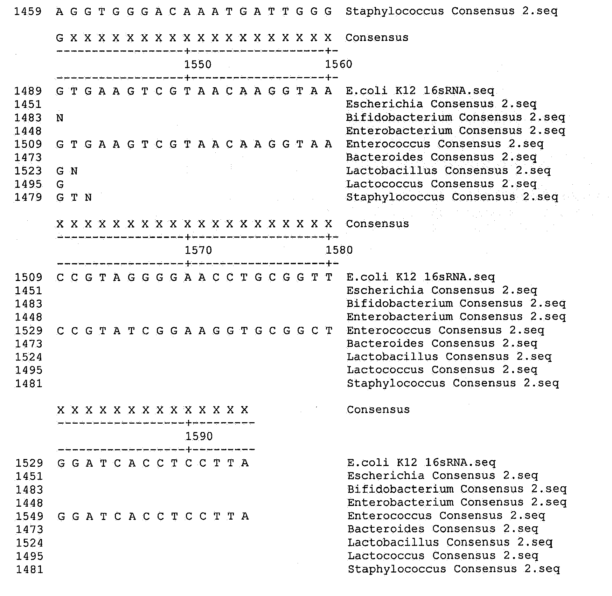

In one aspect the invention provides a method for determining whether a sample comprises nucleic acid of at least one member of Bifidobacteria, comprising administering to nucleic acid of said sample a probe with a length of between 10 and 50 nucleotides, preferably between 13 and 30 nucleotides, comprising a nucleic acid sequence capable of specifically binding to a 16S rRNA nucleic acid sequence corresponding to the nucleic acid sequence located from about nucleotide position 1104 to about 1175 in the Bifidobacterium consensus sequence depicted in Figure 1, and/or capable of specifically binding to a 16S rRNA nucleic acid sequence corresponding to the nucleic acid sequence located from about nucleotide position 1162 to about 1175 in the general consensus sequence depicted in Figure 1, and
- determining whether said probe specifically binds nucleic acid of said sample

Such probe is preferably capable of specifically hybridizing with the above mentioned 16S rRNA nucleic acid sequences under stringent conditions.

Ribosomal RNA (rRNA) is the central component of the ribosome, the protein manufacturing machinery of all living cells. The small 30S subunit of prokaryotic ribosomes comprises 16S rRNA and various proteins. The 16S rRNA is important for subunit association and translational accuracy. Figure 1 shows an alignment of (consensus) 16S rRNA nucleic acid sequences of eight bacterial genera. Since the 16S rRNA nucleic acid sequences are highly conserved, the corresponding positions of 16S rRNA nucleic acid sequences in other *Bifidobacterium* strains are easily assessed based on homology.

A 16S rRNA nucleic acid sequence is defined herein as any kind of (natural or non-natural) nucleic acid sequence corresponding to a 16S rRNA sequence. A nucleic acid sequence corresponds to a 16S rRNA sequence when said nucleic acid sequence comprises a sequence which is capable of specifically binding to a complementary sequence of said 16S rRNA sequence. A nucleic acid sequence also corresponds to a 16S rRNA sequence when said nucleic acid sequence comprises a sequence which is capable of specifically binding to a 16S rRNA sequence, meaning that said nucleic acid sequence comprises a sequence which is complementary to said 16S rRNA sequence. The term "16S rRNA nucleic acid sequence" for instance encompasses a DNA sequence comprising a sequence which is capable of specifically binding to a 16S rRNA sequence. The term "16S rRNA nucleic acid sequence" also encompasses a DNA sequence comprising a sequence which is identical to a 16S rRNA sequence (except, of course, the fact that DNA comprises thymine instead of uracil). The term "16S rRNA nucleic acid sequence" hence encompasses a 16S rDNA sequence. As used herein, the term "16S rRNA sequence" means any kind of 16S rRNA nucleic acid sequence.

According to the invention, a probe capable of specifically hybridizing to a 16S rRNA nucleic acid sequence corresponding to the nucleic acid sequence located from about nucleotide position 1104 to about 1175 in the Bifidobacterium consensus sequence depicted in Figure 1, and/or capable of specifically binding to a 16S rRNA nucleic acid sequence corresponding to the nucleic acid sequence located from about nucleotide position 1162 to about 1175 in the general consensus sequence depicted in Figure 1, is particularly suitable for detecting Bifidobacteria because such probe is capable of detecting nucleic acids of a wide variety of *Bifidobacterium* strains. Using sequence alignments, a skilled person is well capable of determining which 16S rRNA sequences of other *Bifidobacterium* strains correspond to the above mentioned 16S rRNA sequences. Most preferably a probe according to the invention is provided which is capable of specifically hybridizing to a 16S rRNA nucleic acid sequence located from about nucleotide position 1104 to about 1175 in the Bifidobacterium consensus sequence depicted in Figure 1.

A first nucleic acid molecule specifically hybridizes to a second nucleic acid molecule if said first nucleic acid molecule comprises a sequence of at least four, preferably at least five, more preferably at least six nucleotides which is complementary to at least part of a sequence of said second nucleic acid molecule. As is well known by the skilled person, adenine (A) is complementary to thymine (T) and uracil (U). Guanine (G) is complementary to cytosine (C). The term "specifically hybridizing" or "specifically binding" of a nucleic acid sequence to another nucleic acid sequence therefore means a binding event resulting from hybridization of complementary nucleic acid sequences. The terms do not encompass non-specific sticking of nucleic acid molecules, which is not due to a complementary sequence. However, the terms encompass a binding event of two nucleic acid molecules under mild conditions, which nucleic acid molecules comprise sequences that are less than 100% but more than 65% complementary to each other. Two nucleic acid sequences that are less than 100% but more than 65% complementary to each other are nevertheless capable of hybridizing under mild conditions such as for instance a low temperature. Each of said two nucleic acid molecules preferably comprises a sequence that is at least 70%, preferably at least 75%, more preferably at least 80%, more preferably at least 85% complementary to a sequence of the other nucleic acid molecule.

If stringent conditions are used, however, two nucleic acid molecules only bind if they comprise a sequence which is at least 85%, preferably at least 90%, complementary to a sequence of the other nucleic acid molecule. Stringent conditions are therefore defined herein as conditions where hybridization of nucleic acid molecules only takes place if said nucleic acid molecules comprise a sequence which is at least 85%, preferably at least 90%, complementary to a sequence of the other nucleic acid molecule.

A nucleic acid sequence as used herein for instance comprises naturally occurring nucleic acids, such as DNA, RNA or a DNA/RNA helix, modified nucleic acid sequences and/or nucleic acid derivatives such as for instance peptide nucleic acid (PNA). Possible bases of a nucleic acid sequence according to the invention comprise naturally occurring purines and/or pyrimidines, such as adenine, thymine, cytosine, guanine and uracil, as well as modified bases such as for instance inosine.

One embodiment of the invention provides a method for determining whether a sample comprises nucleic acid of at least one member of Bifidobacteria, comprising:
- administering to nucleic acid of said sample a probe with a length of between 10 and 50 nucleotides, preferably between 13 and 30 nucleotides, comprising a nucleic acid sequence which is at least 70% homologous to at least part of the sequence TCGCCGCATGTTGC, said part having at least 10 nucleotides, and
- determining whether said probe specifically binds nucleic acid of said sample.

Such probe is particularly well capable of detecting a wide variety of Bifidobacteria. Said probe preferably comprises a nucleic acid sequence which is at least 75%, more preferably at least 80%, more preferably at least 85%, more preferably at least 90%, most preferably at least 95% homologous to at least part of the sequence TCGCCGCATGTTGC, said part having at least 10 nucleotides, preferably at least 12 nucleotides. The higher the homology, the more *Bifidobacterium* strains are detectable with said probe. In one particularly preferred embodiment a probe consisting of the sequence TCGCCGCATGTTGC is used.

Another embodiment of the invention provides a method for determining whether a sample comprises nucleic acid of at least one member of Bifidobacteria, comprising:
- administering to nucleic acid of said sample a probe with a length of between 10 and 50 nucleotides, preferably between 13 and 30 nucleotides, comprising a nucleic acid sequence which is at least 70% homologous to at least part of the sequence TCGCCCCGTGTTGC, said part having at least 10 nucleotides, and
- determining whether said probe specifically binds nucleic acid of said sample.

Such probe is particularly well capable of detecting a wide variety of Bifidobacteria. Said probe preferably comprises a nucleic acid sequence which is at least 75%, more preferably at least 80%, more preferably at least 85%, more preferably at least 90%, most preferably at least 95% homologous to at least part of the sequence TCGCCCCGTGTTGC, said part having at least 10 nucleotides, preferably at least 12 nucleotides. The higher the homology, the more *Bifidobacterium* strains are detectable with said probe. In one particularly preferred embodiment a probe consisting of the sequence TCGCCCCGTGTTGC is used.

The present invention provides the insight that even more different *Bifidobacterium* strains are detectable if the above mentioned probes are combined. In a preferred embodiment a first probe with a length of between 10 and 50 nucleotides, preferably between 13 and 30 nucleotides, comprising a nucleic acid sequence which is at least 70% homologous to at least part of the sequence TCGCCGCATGTTGC is used in combination with a second probe with a length of between 10 and 50 nucleotides, preferably between 13 and 30 nucleotides, comprising a nucleic acid sequence which is at least 70% homologous to at least part of the sequence TCGCCCCGTGTTGC, said part having at least 10 nucleotides. Said first probe preferably comprises a nucleic acid sequence which is at least 75%, more preferably at least 80%, more preferably at least 85%, more preferably at least 90%, most preferably at least 95% homologous to at least part of the sequence TCGCCGCATGTTGC, said part having at least 10 nucleotides, preferably at least 12 nucleotides. Said second probe preferably comprises a nucleic acid sequence which is at least 75%, more preferably at least 80%, more preferably at least 85%, more preferably at least 90%, most preferably at least 95% homologous to at least part of the sequence TCGCCCCGTGTTGC, said part having at least 10 nucleotides, preferably at least 12 nucleotides. The higher the homology, the more *Bifidobacterium* strains are detectable with said probes. Whereas currently known *Bifidobacterium-*specific probes involve the risk of false negative results, meaning that one or more *Bifidobacterium* strains are not detected, this risk is particularly well diminished if a combination of at least two of the above mentioned probes are used. With a combination of two *Bifidobacterium-specific* probes according to the present invention, at least 29 different Bifidobacterial species are detectable. Hence, a combination of at least two *Bifidobacterium-*specific probes according to the invention provides a preferred tool for detection of Bifidobacteria. Further provided is therefore a method for determining whether a sample comprises nucleic acid of at least one member of *Bifidobacterium,* comprising:
- administering to nucleic acid of said sample a probe with a length of between 10 and 50 nucleotides, preferably between 13 and 30 nucleotides, comprising a nucleic acid sequence which is at least 70%, preferably at least 75%, more preferably at least 80%, more preferably at least 85%, more preferably at least 90%, most preferably at least 95% homologous to at least part of the sequence TCGCCGCATGTTGC, said part having at least 10 nucleotides,
- administering to nucleic acid of said sample a probe with a length of between 10 and 50 nucleotides, preferably between 13 and 30 nucleotides, comprising a nucleic acid sequence which is at least 70%, preferably at least 75%, more preferably at least 80%, more preferably at least 85%, more preferably at least 90%, most preferably at least 95% homologous to at least part of the sequence TCGCCCCGTGTTGC, said part having at least 10 nucleotides, and
- determining whether at least one of said probes specifically binds nucleic acid of said sample. In one particularly preferred embodiment a probe consisting of the sequence TCGCCGCATGTTGC in combination with a probe consisting of the sequence TCGCCCCGTGTTGC is used.

In order to detect a low amount of nucleic acid of interest in a sample, a nucleic acid amplification reaction is often performed. During such amplification reaction the amount of nucleic acid is increased. A nucleic acid amplification reaction comprises any kind of reaction wherein a nucleic acid sequence is duplicated at least one time. Preferred amplification reactions comprise (reverse transcriptase)-polymerase chain reaction ((RT)-PCR), nucleic acid sequence based amplification (NASBA), strand displacement amplification (SDA) and transcription mediated amplification (SDA). Methods of performing a nucleic acid amplification reaction are well known in the art. A PCR protocol is for instance described in (Sambrook, J., Fritsch, E.F., and Maniatis, T. 1989. Molecular cloning: a laboratory manual. Cold Spring Harbor laboratory Press, Cold Spring Harbor, N:Y.). (Called herein Sambrook et al (1989)).

A preferred embodiment thus provides a method according to the invention, wherein nucleic acid of said sample is subjected to a nucleic acid amplification reaction. In one embodiment nucleic acid is firstly amplified, where after a probe according to the invention, or preferably a combination of at least two probes according to the invention, is administered to the amplified nucleic acid in order to determine whether *Bifidobacterium* nucleic acid is present. Amplified nucleic acid is incubated with at least one probe, unbound probe is preferably washed away and bound probe is visualized, for instance using a visible label and/or an enzyme reaction. These methods are well known and need no further explanation here. Preferably however real time monitoring is performed, for instance using a Taqman or beacon probe. A Taqman probe comprises an oligonucleotide, complementary to a target sequence, which is labeled with a 5'-reporter dye and a 3'-quencher dye. When both dyes are attached to the probe the reporter dye cannot be detected. A TaqMan probe hybridizes to a target nucleic acid sequence. During a nucleic acid amplification reaction the TaqMan probe is hydrolyzed, resulting in separation of the reporter dye and quencher dyes. This, in turn, results in increased fluorescence of the reporter. Increase in fluorescence is monitored over time. Many well known alternative methods for detection of amplified nucleic acid are available in the art

In order to specifically amplify Bifidobacterial nucleic acid, a primer capable of specifically hybridizing to Bifidobacterial nucleic acid is preferably used. Said primer is capable of binding a higher number of different *Bifidobacterium* nucleic acid sequences, as compared to the number of different non-*Bifidobacterium* nucleic acid sequences that said primer is capable of binding to. Such primer is called herein a *Bifidobacterium*-specific primer. Said primer is preferably capable of binding less than ten, preferably less than eight, more preferably less than six different non-*Bifidobacterium* nucleic acid sequences. Most preferably said primer is only capable of specifically binding to nucleic acid of Bifidobacterial strains. This way, amplification of nucleic acid of other species is at least in part avoided. A method according to the invention comprising subjecting nucleic acid of a sample to a nucleic acid amplification reaction using at least one *Bifidobacterium*-specific primer is therefore also provided.

In one preferred embodiment nucleic acid is amplified with a primer with a length of between 9 and 50 nucleotides, preferably with a length of between 9 and 40 nucleotides, more preferably with a length of between 15 and 25 nucleotides, which primer is capable of specifically hybridizing to a 16S rRNA nucleic acid sequence corresponding to the nucleic acid sequence located from about nucleotide position 969 to about 989 in the Bifidobacterium consensus sequence depicted in Figure 1, and/or capable of specifically hybridizing to a 16S rRNA nucleic acid sequence corresponding to the nucleic acid sequence located from about nucleotide position 1023 to about 1043 in the general consensus sequence depicted in Figure 1. Said primer preferably comprises a nucleic acid sequence which is at least 70% homologous to at least part of the sequence CCTTACCTGGGCTTGACATGT, said part having at least 10 nucleotides. Preferably, said part comprises at least 15 nucleotides. Said primer preferably comprises a nucleic acid sequence which is at least 75%, more preferably at least 80%, more preferably at least 85%, more preferably at least 90%, most preferably at least 95% homologous to at least part of the sequence CCTTACCTGGGCTTGACATGT, said part having at least 10 nucleotides. In one preferred embodiment a primer consisting of the sequence CCTTACCTGGGCTTGACATGT is used.

In another preferred embodiment nucleic acid is amplified using a primer with a length of between 9 and 50 nucleotides, preferably with a length of between 9 and 40 nucleotides, more preferably with a length of between 15 and 25 nucleotides, which primer is capable of specifically hybridizing to a 16S rRNA nucleic acid sequence corresponding to the nucleic acid sequence located from about nucleotide position 1187 to about 1207 in the Bifidobacterium consensus sequence depicted in Figure 1, and/or capable of specifically hybridizing to a 16S rRNA nucleic acid sequence corresponding to the nucleic acid sequence located from about nucleotide position 1245 to about 1265 in the general consensus sequence depicted in Figure 1. Said primer preferably comprises a nucleic acid sequence which is at least 70% homologous to at least part of the sequence GACGTAAGGGGCATGATGATC, said part having at least 10 nucleotides, preferably at least 15 nucleotides. Said primer preferably comprises a nucleic acid sequence which is at least 75%, more preferably at least 80%, more preferably at least 85%, more preferably at least 90%, most preferably at least 95% homologous to at least part of the sequence GACGTAAGGGGCATGATGATC, said part having at least 10 nucleotides. In one preferred embodiment a primer consisting of the sequence GACGTAAGGGGCATGATGATC is used.

A primer according to the invention is preferred because it is suitable for amplifying nucleic acid of a wide variety of (at least 29) different Bifidobacteria species while amplification of non-Bifidobacterial nucleic acid is at least in part avoided.

In a particularly preferred embodiment, two of the above mentioned primers according to the invention are combined. A primer with a length of between 9 and 50 nucleotides, preferably with a length of between 9 and 40 nucleotides, more preferably with a length of between 15 and 25 nucleotides, comprising a nucleic acid sequence which is at least 70%, preferably at least 75%, more preferably at least 80%, more preferably at least 85%, more preferably at least 90%, most preferably at least 95% homologous to at least part of the sequence CCTTACCTGGGCTTGACATGT, said part having at least 10 nucleotides, is preferably used as a forward primer while a primer with a length of between 9 and 50 nucleotides, preferably with a length of between 9 and 40 nucleotides, more preferably with a length of between 15 and 25 nucleotides, comprising a nucleic acid sequence which is at least 70%, preferably at least 75%, more preferably at least 80%, more preferably at least 85%, more preferably at least 90%, most preferably at least 95% homologous to at least part of the sequence GACGTAAGGGGCATGrATGATC, said part having at least 10 nucleotides, is preferably used as a reverse primer. This combination of primers according to the invention is particularly suitable for specifically amplifying a wide variety of Bifidobacterial nucleic acid, while at least in part avoiding amplification of non-Bifidobacterial nucleic acid. In one particularly preferred embodiment a primer consisting of the sequence CCTTACCTGGGCTTGACATGT in combination with a primer consisting of the sequence GACGTAAGGGGCATGATGATC is used.

For some applications detection of the presence or absence of Bifidobacterial nucleic acid is sufficient, for instance if contamination of a supposedly sterile sample is examined. In other applications however, quantification of the amount of Bifidobacterial nucleic acid is preferred. For instance, the amount of Bifidobacterial nucleic acid in a sample derived from an individual's gastrointestinal tract is preferably quantified because a sufficiently high, natural amount of Bifidobacteria is important for the well-being of individuals whereas a diminished amount of Bifidobacteria has an influence on the general health status and often coincides with an increase in susceptibility to gastroenterological infections and chronic diseases like colon cancer. A method according to the invention which further comprises quantifying nucleic acid of at least one member of Bifidobacteria is therefore also provided herewith. Quantitation of nucleic acid is for instance performed using quantitative nucleic acid amplification, which is well known in the art. In one embodiment known amounts of nucleic acid of interest are amplified and visualised beforehand. The obtained values are plotted in order to obtain a reference curve. Subsequently, an unknown amount of nucleic acid is amplified and visualised, where after the obtained value is compared with said reference curve in order to establish the (original) amount of said nucleic acid of interest. In another embodiment real time quantitative nucleic acid amplification is used with Taqman or beacon probes. According to this embodiment an amplification plot is established in which a fluorescence signal is plotted versus the cycle number. In the initial cycles of a nucleic acid amplification reaction, there is generally little change in the fluorescence signal. This defines the baseline for the amplification plot. An increase in fluorescence above the baseline implies detection of accumulated nucleic acid amplification product. A fixed fluorescence threshold is preferably set above the baseline. According to this embodiment a parameter Ct is defined as the fractional cycle number at which the fluorescence passes the fixed threshold. Quantitation of the amount of target nucleic acid in unknown samples is subsequently preferably accomplished by measuring the Ct and using said standard curve to determine the starting copy number.

Nucleic acid is also quantified in various alternative ways, which are known in the art and need no further explanation.

In one embodiment the presence and/or amount of total Bifidobacterial nucleic acid content in a sample is investigated. Additionally, or alternatively, nucleic acid of at least one member of Bifidobacteria is identified. This embodiment is particularly suitable in situations wherein the presence of at least one particular Bifidobacterial strain is investigated, for instance when nucleic acid of an unknown species (for instance present in a food product) is amplified and identified, or when nucleic acid of a pool of Bifidobacteria (for instance present in faeces) is amplified after which the different species are identified. In one embodiment a method according to the present invention is performed with a first probe according to the invention which is at least 70% homologous to at least part of the sequence TCGCCGCATGTTGC and a second probe according to the invention which is at least 70% homologous to at least part of the sequence TCGCCCCGTGTTGC, after which one of the probes is used in order to provide more information about the presence or absence of individual species.

A method according to the invention is, amongst other things, particularly suitable for investigating the presence and/or amount of *Bifidobacterium* species in the gastrointestinal tract. A faeces sample is particularly well suitable for this purpose. Further provided is therefore a method according to the invention, wherein said nucleic acid is obtained from a faeces sample.

If a member of the *Bifidobacterium* genus is present in a sample, it is particularly well detected with a probe according to the present invention. Such probe is also suitable for isolating nucleic acid of a *Bifidobacterium* member. Detection and/or isolation of *Bifidobacterium* nucleic acid is for instance performed using a column or dipstick with at least one probe according to the invention. Such column or dipstick is incubated with a sample, where after unbound components are washed away and bound nucleic acid is detected and/or isolated. Various alternative ways of detecting and/or isolating nucleic acid using a probe sequence are available in the art, which are also applicable. Further provided is therefore a method for detecting and/or isolating nucleic acid of at least one member of Bifidobacteria, comprising administering a probe with a length of between 10 and 50 nucleotides, preferably between 13 and 30 nucleotides, comprising a nucleic acid sequence which is at least 70% homologous to at least part of the sequence TCGCCGCATGTTGC, said part having at least 10 nucleotides, to nucleic acid of at least one member of Bifidobacteria. Said probe preferably comprises a nucleic acid sequence which is at least 75%, more preferably at least 80%, more preferably at least 85%, more preferably at least 90%, most preferably at least 95% homologous to at least part of the sequence TCGCCGCATGTTGC, said part having at least 10 nucleotides, preferably at least 12 nucleotides. The higher the homology, the more *Bifidobacterium* strains are detectable with said probe. Most preferably, said probe consists of the sequence TCGCCGCATGTTGC.

Further provided is a method for detecting and/or isolating nucleic acid of at least one member of Bifidobacteria, comprising administering a probe with a length of between 10 and 50 nucleotides, preferably between 13 and 30 nucleotides, comprising a nucleic acid sequence which is at least 70% homologous to at least part of the sequence TCGCCCCGTGTTGC, said part having at least 10 nucleotides, to nucleic acid of at least one member of Bifidobacteria. Said probe preferably comprises a nucleic acid sequence which is at least 75%, more preferably at least 80%, more preferably at least 85%, more preferably at least 90%, most preferably at least 95% homologous to at least part of the sequence TCGCCCCGTGTTGC, said part having at least 10 nucleotides, preferably at least 12 nucleotides. The higher the homology, the more *Bifidobacterium* strains are detectable with said probe. Most preferably, said probe consists of the sequence TCGCCCCGTGTTGC.

As explained before, the present invention provides the insight that a combination of at least two different probes according to the present invention is preferred because even more different *Bifidobacterium* strains are detectable with said combination. Hence, in a particularly preferred embodiment, the above mentioned probe comprising a nucleic acid sequence which is at least 70% homologous to at least part of the sequence TCGCCGCATGTTGC is combined with the above mentioned probe comprising a nucleic acid sequence which is at least 70% homologous to at least part of the sequence TCGCCCCGTGTTGC. In one particularly preferred embodiment a probe consisting of the sequence TCGCCGCATGTTGC in combination with a probe consisting of the sequence TCGCCCCGTGTTGC is used.

In order to detect a low amount of nucleic acid of interest in a sample, a nucleic acid amplification reaction is often performed. A primer according to the present invention capable of specifically hybridizing to Bifidobacterial nucleic acid is preferably used so that Bifidobacterial nucleic acid is specifically amplified while amplification of nucleic acid of other genera is at least in part avoided.

A method for subjecting nucleic acid to a nucleic acid amplification reaction comprising providing said nucleic acid with at least one primer and performing a nucleic acid amplification reaction, wherein said nucleic acid is provided with a primer with a length of between 9 and 50 nucleotides, preferably with a length of between 9 and 40 nucleotides, more preferably with a length of between 15 and 25 nucleotides, which primer is capable of specifically hybridizing to a 16S rRNA nucleic acid sequence corresponding to the nucleic acid sequence located from about nucleotide position 969 to about 989 in the Bifidobacterium consensus sequence depicted in Figure 1, and/or capable of specifically hybridizing to a 16S rRNA nucleic acid sequence corresponding to the nucleic acid sequence located from about nucleotide position 1023 to about 1043 in the general consensus sequence depicted in Figure 1 is therefore also provided. Said primer preferably comprises a nucleic acid sequence which is at least 70% homologous to at least part of the sequence CCTTACCTGGGCTTGACATGT, said part having at least 10 nucleotides. such primer is capable of hybridizing to nucleic acid of a wide variety of Bifidobacterial species, while amplification of non-Bifidobacterial nucleic acid is at least in part avoided.

Further provided is a method for subjecting nucleic acid to a nucleic acid amplification reaction comprising providing said nucleic acid with at least one primer and performing a nucleic acid amplification reaction, wherein said nucleic acid is provided with a primer with a length of between 9 and 50 nucleotides, preferably with a length of between 9 and 40 nucleotides, more preferably with a length of between 15 and 25 nucleotides, which primer is capable of specifically hybridizing to a 16S rRNA nucleic acid sequence corresponding to the nucleic acid sequence located from about nucleotide position 1187 to about 1207 in the Bifidobacterium consensus sequence depicted in Figure 1, and/or capable of specifically hybridizing to a 16S rRNA nucleic acid sequence corresponding to the nucleic acid sequence located from about nucleotide position 1245 to about 1265 in the general consensus sequence depicted in Figure 1. Said primer preferably comprises a nucleic acid sequence which is at least 70% homologous to at least part of the sequence GACGTAAGGGGCATGATGATC, said part having at least 10 nucleotides. Such primer is capable of hybridizing to nucleic acid of a wide variety of Bifidobacterial species, while amplification of non-Bifidobacterial nucleic acid is at least in part avoided. Further provided is therefore a method for subjecting nucleic acid to a nucleic acid amplification reaction, comprising providing said nucleic acid with a primer with a length of between 9 and 50 nucleotides, preferably with a length of between 9 and 40 nucleotides, more preferably with a length of between 15 and 25 nucleotides, comprising a nucleic acid sequence which is at least 70%, preferably at least 75%, more preferably at least 80%, more preferably at least 85%, more preferably at least 90%, most preferably at least 95% homologous to at least part of the sequence CCTTACCTGGGCTTGACATGT or GACGTAAGGGGCATGATGATC, said part having at least 10 nucleotides, preferably at least 15 nucleotides. Nucleic acid of a sample is preferably provided with a combination of a forward primer and a reverse primer, wherein the forward primer is a primer with a length of between 9 and 50 nucleotides, preferably with a length of between 9 and 40 nucleotides, more preferably with a length of between 15 and 25 nucleotides, comprising a nucleic acid sequence which is at least 70%, preferably at least 75%, more preferably at least 80%, more preferably at least 85%, more preferably at least 90%, most preferably at least 95% homologous to at least part of the sequence CCTTACCTGGGCTTGACATGT, said part having at least 10 nucleotides, and wherein the reverse primer is a primer with a length of between 9 and 50 nucleotides, preferably with a length of between 9 and 40 nucleotides, more preferably with a length of between 15 and 25 nucleotides, comprising a nucleic acid sequence which is at least 70%, preferably at least 75%, more preferably at least 80%, more preferably at least 85%, more preferably at least 90%, most preferably at least 95% homologous to at least part of the sequence GACGTAAGGGGCATGATGATC, said part having at least 10 nucleotides. This combination of primers according to the invention is particularly suitable for specifically amplifying a wide variety of Bifidobacterial nucleic acid, while at least in part avoiding amplification of non-Bifidobacterial nucleic acid. In one particularly preferred embodiment a primer consisting of the sequence CCTTACCTGGGCTTGACATGT in combination with a primer consisting of the sequence GACGTAAGGGGCATGATGATC is used.

Further provided are probes and primers according to the present invention, as well as kits comprising these primers and/or probes, which are particularly suitable for detecting, amplifying, quantifying and/or isolating Bifidobacterial nucleic acid. A nucleic acid sequence with a length of between 10 and 50 nucleotides, preferably between 13 and 30 nucleotides, comprising a sequence which is at least 70%, preferably at least 75%, more preferably at least 80%, more preferably at least 85%, more preferably at least 90%, most preferably at least 95% homologous to at least part of the sequence TCGCCGCATGTTGC, said part having at least 10 nucleotides, is therefore also herewith provided. Such nucleic acid sequence is particularly suitable for use as a probe. Said nucleic acid preferably consists of the sequence TCGCCGCATGTTGC. A nucleic acid sequence with a length of between 10 and 50 nucleotides, preferably between 13 and 30 nucleotides, comprising a sequence which is at least 70%, preferably at least 75%, more preferably at least 80%, more preferably at least 85%, more preferably at least 90%, most preferably at least 95% homologous to at least part of the sequence TCGCCCCGTGTTGC, said part having at least 10 nucleotides, is also herewith provided. Such nucleic acid sequence is also particularly suitable for use as a probe. Said nucleic acid preferably consists of the sequence TCGCCCCGTGTTGC. A nucleic acid sequence with a length of between 9 and 50 nucleotides, preferably with a length of between 9 and 40 nucleotides, more preferably with a length of between 15 and 25 nucleotides, comprising a sequence which is at least 70%, preferably at least 75%, more preferably at least 80%, more preferably at least 85%, more preferably at least 90%, most preferably at least 95% homologous to at least part of the sequence CCTTACCTGGGCTTGACATGT, said part having at least 10 nucleotides, preferably at least 15 nucleotides is also herewith provided. Such nucleic acid is particularly suitable for use as a forward primer for specifically amplifying Bifidobacterial nucleic acid. Said nucleic acid preferably consists of the sequence CCTTACCTGGGCTTGACATGT. The invention furthermore provides a nucleic acid sequence with a length of between 9 and 50 nucleotides, preferably with a length of between 9 and 40 nucleotides, more preferably with a length of between 15 and 25 nucleotides, comprising a sequence which is at least 70%, preferably at least 75%, more preferably at least 80%, more preferably at least 85%, more preferably at least 90%, most preferably at least 95% homologous to at least part of the sequence GACGTAAGGGGCATGATGATC, said part having at least 10 nucleotides, preferably at least 15 nucleotides. Such nucleic acid is particularly suitable for use as a reverse primer for specifically amplifying Bifidobacterial nucleic acid. Said nucleic acid preferably consists of the sequence GACGTAAGGGGCATGATGATC.

A particularly preferred embodiment provides a primer pair consisting of the above mentioned forward primer and the above mentioned reverse primer. Further provided is therefore a primer pair consisting of:
- a primer with a length of between 9 and 50 nucleotides, preferably with a length of between 9 and 40 nucleotides, more preferably with a length of between 15 and 25 nucleotides, comprising a nucleic acid sequence which is at least 70%, preferably at least 75%, more preferably at least 80%, more preferably at least 85%, more preferably at least 90%, most preferably at least 95% homologous to at least part of the sequence CCTTACCTGGGCTTGACATGT, said part having at least 10 nucleotides, and
- a primer with a length of between 9 and 50 nucleotides, preferably with a length of between 9 and 40 nucleotides, more preferably with a length of between 15 and 25 nucleotides, comprising a nucleic acid sequence which is at least 70%, preferably at least 75%, more preferably at least 80%, more preferably at least 85%, more preferably at least 90%, most preferably at least 95% homologous to at least part of the sequence GACGTAAGGGGCATGATGATC, said part having at least 10 nucleotides.

In one particularly preferred embodiment a primer pair consisting of a primer consisting of the sequence CCTTACCTGGGCTTGACATGT in combination with a primer consisting of the sequence GACGTAAGGGGCATGATGATC is provided.

The invention furthermore provides a kit for detecting Bifidobacterial nucleic acid, comprising at least one probe according to the invention. Said kit preferably comprises a combination of at least two different probes according to the invention, as outlined before. A kit for amplifying Bifidobacterial nucleic acid comprising at least one primer according to the invention is also provided. Said kit preferably comprises a primer pair according to the invention. A kit comprising at least one probe, preferably at least two probes, and at least one primer according to the present invention is also provided.

In one aspect a method according to the invention is used for testing whether a candidate compound is capable of influencing the amount of Bifidobacteria in a population. This is for instance useful for identifying, developing and/or testing probiotics and/or prebiotics. Probiotics and prebiotics are useful for improving and/or restoring a normal healthy equilibrium in an individual's digestive tract. Probiotics are live micro-organisms, such as *Bifidobacterium* species, that are capable of improving the balance of the intestinal microflora. Probiotic bacterial cultures assist the body's naturally occurring flora within the digestive tract to reestablish themselves. Prebiotics are food ingredients that beneficially affect the host by selectively stimulating the growth and/or the activity of a limited number of bacteria in the colon. In order to monitor the effect of a certain probiotic or prebiotic, or in order to screen a candidate compound for beneficial activity, the effects upon the presence and/or amount of Bifidobacteria is preferably assessed since Bifidobacteria are known to contribute to the well-being of individuals. The effect of a (candidate) probiotic and/or (candidate) prebiotic is preferably assessed by determining the presence and/or amount of Bifidobacteria in a sample from an individual that has been provided with said (candidate) probiotic and/or (candidate) prebiotic, using a method according to the present invention. Preferably, the presence and/or amount of Bifidobacteria in a first sample of an individual is investigated with a method according to the invention before said individual has been provided with a (candidate) probiotic and/or (candidate) prebiotic. Subsequently, the presence and/or amount of Bifidobacteria in the same kind of sample (called herein a second sample) of said individual is investigated with a method according to the invention, which second sample is obtained after said individual has been provided with said (candidate) probiotic and/or (candidate) prebiotic. Differences in the amount or concentration of Bifidobacteria in said first and second sample is indicative for an effect of said (candidate) probiotic and/or (candidate) prebiotic. Further provided is therefore a method for determining whether a candidate compound is capable of influencing the amount of Bifidobacteria in a population, comprising:
- providing said population with said candidate compound, and
- determining whether nucleic acid of at least one member of Bifidobacteria is present in a sample of said population, using a method according to the invention. Said population preferably comprises a population of the gastrointestinal tract. If Bifidobacterial nucleic acid is present, nucleic acid of at least one member of *Bifidobacterium* is preferably quantified because the amount of Bifidobacteria is indicative for the health status of an individual. Preferably, all Bifidobacterial nucleic acid present in said sample is quantified. The presence and/or amount (concentration) of Bifidobacterial nucleic acid in said sample is preferably compared with the presence and/or amount (concentration) of Bifidobacterial nucleic acid in a reference sample of said population, obtained before said population had been provided with said (candidate) compound.

If a (candidate) compound appears to be capable of beneficially influencing the amount of Bifidobacteria in said population, it is preferably selected and/or isolated. A method according to the invention, further comprising selecting and/or isolating a candidate compound capable of beneficially influencing the amount of Bifidobacteria in said population is therefore also provided. Preferably a candidate compound is selected and/or isolated which is capable of increasing the amount of Bifidobacteria in said population. Said selected and/or isolated compound is preferably incorporated into a food product in order to improve health. Said selected and/or isolated compound is preferably incorporated into a dairy product. A food product capable of beneficially influencing the amount of Bifidobacteria, obtainable by a method according to the invention is therefore also herewith provided. Said food product preferably comprises a dairy product.

The invention is further explained in the following examples. These examples do not limit the scope of the invention, but merely serve to clarify the invention.

### Examples

### Example 1

The following primers and probe were used:
prF21bifido CCTTACCTGGGCTTGACATGT
prR21bifido GACGTAAGGGGCATGATGATC
probe A 6-fam-TCGCCCCGTGTTGC-mgb

Twenty-five µl of reaction mixture was composed of 12,5 µl 2x Universal PCR Master Mix (TaqMan, Applied Biosystems), 0,1 µM of each primer, 0,25 µM probe and 5 µl of template DNA. PCR was carried out for 40 cycles of 95 °C for 15 sec and 60 °C for 60 sec.

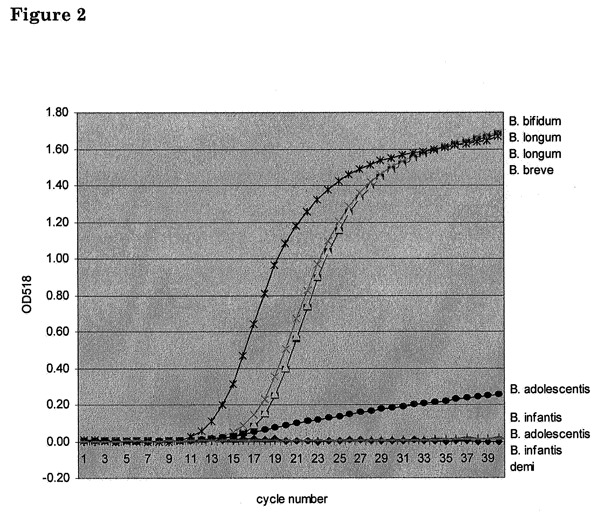

The results are shown in Figure 2.

The presence of many Bifidobacterium species is demonstrated.

### Example 2

The following primers and probe were used:
prF21bifido CCTTACCTGGGCTTGACATGT
prR21bifido GACGTAAGGGGCATGATGATC
probe B 6-fam-TCGCCGCATGTTGC-mgb

Twenty-five µl of reaction mixture was composed of 12,5 µl 2x Universal PCR Master Mix (TaqMan, Applied Biosystems), 0,1 µM of each primer, 0,25 µM probe and 5 µl of template DNA. PCR was carried out for 40 cycles of 95 °C for 15 sec and 60 °C for 60 sec.

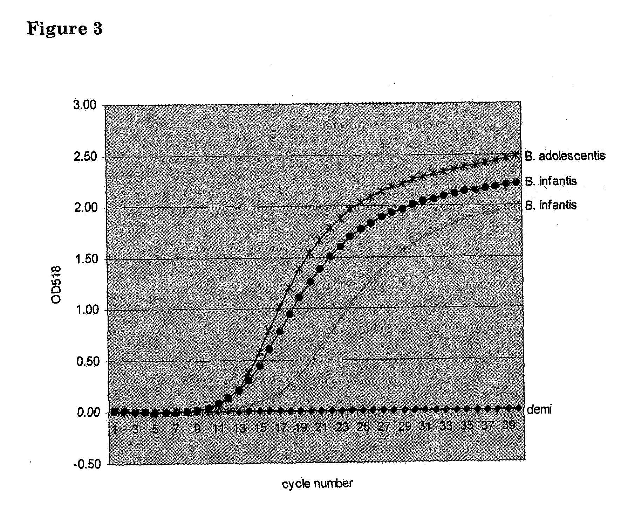

The results are shown in Figure 3.

It is clear that the presence of B. adolescentis and B.infantis is now demonstrated. A combination of Probe A and Probe B is therefore preferred.

### Example 3

A primer consisting of the sequence CCTTACCTGGGCTTGACATGT in combination with a primer consisting of the sequence GACGTAAGGGGCATGATGATC was used.
By using this combination of primers it was tested whether Vivinal galactooligosaccharides exerts a prebiotic effect, i.e. in beneficially affecting the host by selectively stimulating the growth and/or activity of one or a limited number of bacterial species already resident in the colon, and thus attempt to improve host health.

### In vitro fermentation

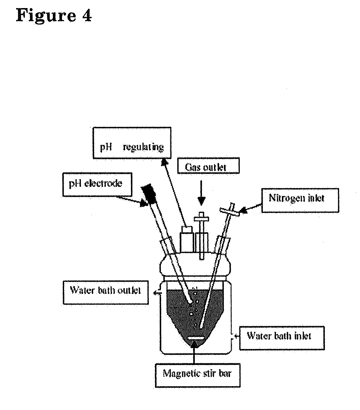

Chemostat nutrient media was prepared containing: Peptone water 2g/l (Oxoid, Basingstoke, UK), yeast extract 2g/l (Oxoid), NaCl 0.1g/l (Fisher, Loughborough, UK), K2HPO4.3H2O 0.05g/l (BDH, Poole, UK), KH2PO4 0.04g/l (BDH), MgSO4.7H2O 0.01g/l (Sigma), CaC12.2H2O 0.005g/l (BDH), NaHCO3 2g/l (Sigma), Tween 80 (BDH) 2ml, Hemin 0.005g/l, Vitamin K1 10µl (Sigma), Cystiene HCl 0.5g/l (Oxoid), Bile Salts 0.5g/l (Oxoid), Resazurin 4ml/l (Sigma), pH7. Of this chemostat nutrient media was aseptically poured into the sterile batch culture. The media was autoclaved and left overnight with nitrogen pumping through the vessel to provide an anaerobic environment. If the media in the vessels had not reduced over night, cysteine-HCl was added to aid reduction of the media. The pH meters (Electrolab pH controller, Tewksbury, UK) were set to regulate the vessels between pH6.8 and pH7.1 by the addition of 1M HCl or 1M NaOH. The vessels were kept at 37°C, and were stirred using magnetic stir bars. See Figure 4.

The next day, a stool sample was collected from several volunteer, free of known gastrointestinal disorders, between the ages of 25-54. None of the volunteers used had taken antibiotics within a three-month period prior to sampling. Each pH-controlled, stirred batch culture experiment was repeated 3 times with 3 different faecal adult donors. Within 15 min of defecation fecal slurry was prepared from 1 part pooled fecal sample and 9 parts pre-reduced phosphate-buffered saline (PBS: 10% w/w) at pH 7.4. To the vessel containing 180 ml of the chemostat nutrient media and 2 g of galacto-oligosaccharides (corrected for lactose and mono-saccharides), 20 ml of fecal slurry was added. The vessels were left for 24hours, with samples taken at 0, 2, 4, 6 and 24hours. For the isolation of bacterial DNA from faecal samples, the QIAamp DNA Stool Mini Kit from QIAgen was used.

Quantitative PCR was used to determine Bifidobacteria:

The 16 s RNA was amplified by PCR with oligonucleotides prF21bifido and prR21bifido. Twenty-five µl of reaction mixture was composed of 12,5 µl 2x Universal PCR Master Mix (TaqMan, Applied Biosystems), 0,1 µM of each primer, 0,25 µM probe and 5 µl of template DNA. PCR was carried out for 40 cycles of 95°C for 15 sec and 60° C for 60 sec.

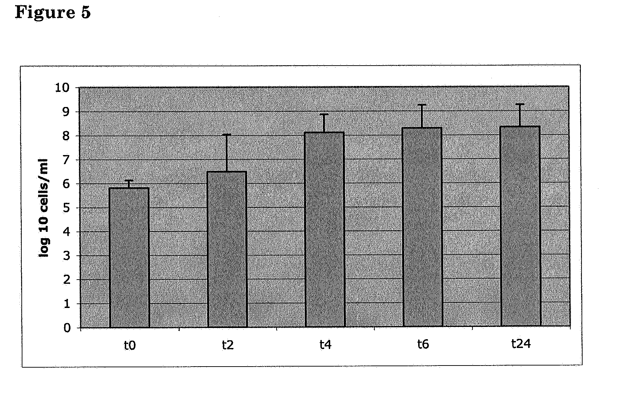

The results are shown in Figure 5: a significant higher numbers of Bifidobacteria were found for V-GOS after 4, 6 and 24 h of fermentation.

### Example 4

### Three-stage continuous culture system

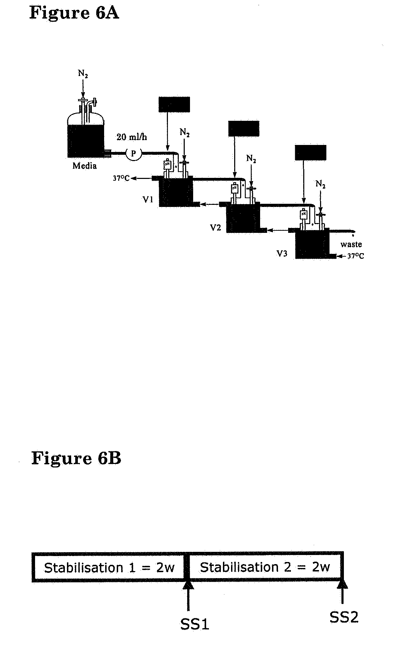

Three autoclaved vessels were connected such that the fermentation media flowed from the media vessel through vessel 1 (volume 280 ml), vessel 2 and vessel 3 (300 ml each) to the waste bottle. See Figure 6A.

Vessels were connected to pH Controller Models 260 (Electrolab) and culture pH maintained at pH 5.5 -5.8 (vessel 1), 6.2 -6.5 (vessel 2), and 6.8 -7.1 (vessel 3). The autoclaved medium was connected to vessel 1 via a pump. Media and fermentation vessels were constantly stirred and kept at 37°C and anaerobic by a constant nitrogen flow.
After filling half of the vessels with medium, roughly the same amount of a freshly prepared faecal slurry was added to each vessel. Three healthy volunteers who had not used antibiotics in the previous three months donated stool samples. Within 30 min following defecation, faecal slurries were prepared from 1 part freshly voided faecal sample and 9 parts pre-reduced phosphate-buffered saline (PBS) (10 % w/v).
The media pump was turned off, and the inoculated vessels incubated without flow for one day. After 24 hours, the medium pump was started again at a flow rate of ca. 20 mL per hour to simulate a retention time of about 44 hours. After two weeks, when an equilibrium or steady state was reached, the carbohydrates in the media were proportionally replaced by the testing substrate. The medium of the last 2 weeks contained 1% substrate. At the time points 0, days 12, 13, 14, 20/21 and 26, 27, 28 samples were taken. Steady state 1 and 2 is calculated as the average of time point 12, 13 and 14, and 26, 27 and 28. See Figure 6B.

For the isolation of bacterial DNA from faecal samples, the QIAamp DNA Stool Mini Kit from QIAgen was used.

16s RNA DNA was amplified by PCR with oligonucleotide prF21bifido and prR21bifido (Bifidobacterium) and with lactobacilli-speeific primers.

### Bifidobacteria-specific primer set (prF21bifido and prR21bifido) prF21bifido: a primer consisting of the sequence

CCTTACCTGGGCTTGACATGT
*prR21bifido:* primer consisting of the sequence GACGTAAGGGGCATGATGATC.

### Lactobacilli-specific primer set:

A primer consisting of the sequence AGTGGAACTCCATGTGTAGCGG in combination with a primer consisting of the sequence
GGTATCTAATCCTGTTCGCTACCCAT.

Quantitative PCR was used to determine Bifidobacteria and Lactobacilli: The 16 s RNA was amplified by PCR with oligonucleotides prF21bifido and prR21bifido. Twenty-five µl of reaction mixture was composed of 12,5 µl 2x Universal PCR Master Mix (TaqMan, Applied Biosystems), 0,1 µM of each primer, 0,25 µM probe and 5 µl of template DNA. PCR was carried out for 40 cycles of 95°C for 15 sec and 60° C for 60 sec.

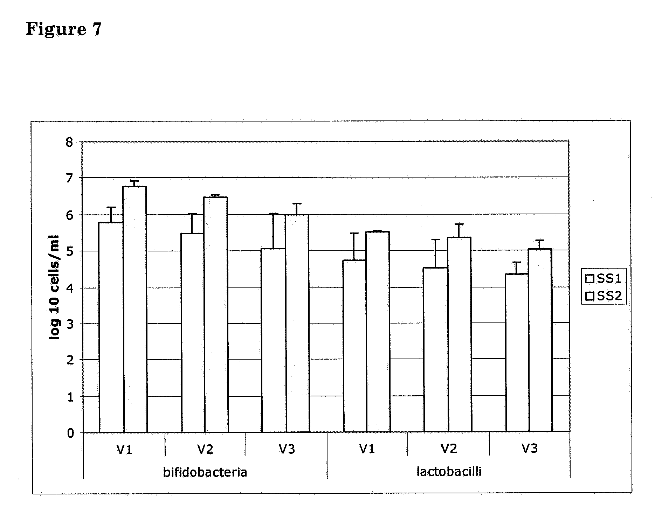

As shown in Figure 7, the addition of Vivinal GOS resulted in significant higher numbers of Bifidobacteria and Lactobacilli (i.e. comparison of steady state 2 (SS2) versus SS1, after correction for donor and vessel and/or substrate).

### Brief description of the drawings

**Figure 1****.** Alignment of 16s rRNA sequences. The 16s rRNA sequence of E. coli K12 represents nucleotide position 223771 - 225312 of the complete genome. Entry: NC_000913 GeneID:944897 16S ribosomal RNA E.coli K12 (Blattner,F.R., Plunkett,G. III, Bloch,C.A., Perna,N.T., Burland,V., Riley,M., Collado-Vides,J., Glasner,J.D., Rode,C.K., Mayhew,G.F., Gregor,J., Davis,N.W., Kirkpatrick,H.A., Goeden,M.A., Rose,D.J., Mau,B. and Shao,Y. (1997). The complete genome sequence of Escherichia coli K-12. Science 277 (5331), 1453-1474).
   Primer sequences are indicated in bold.
**Figure 2****.** Amplification curves of Bifidobacterium strains.
**Figure 3****.** Amplification curves of Bifidobacterium strains.
**Figure 4****.** Experimental set-up of Example 3
**Figure 5****.** Changes in bifidobacteria during fermentation of Vivinal galacto-oligosaccharides
**Figure 6****.** Experimental set-up of Example 4
**Figure 7****.** Microbiota in vessels 1, 2, and 3 of the three-stage continuous culture system before (SS1) and 2 weeks after addition of J-GOS (SS2)

### References

Sambrook, J., Fritsch, E.F., and Maniatis, T. 1989. Molecular cloning: a laboratory manual. Cold Spring Harbor laboratory Press, Cold Spring Harbor, N.Y.). (Called herein Sambrook et al (1989)

## Claims

1. A method for determining whether a sample comprises nucleic acid of at least one member of Bifidobacteria, comprising:
- administering to nucleic acid of said sample a probe with a length of between 10 and 50 nucleotides, preferably between 13 and 30 nucleotides, comprising a nucleic acid sequence capable of specifically binding to a 16S rRNA nucleic acid sequence corresponding to the nucleic acid sequence located from about nucleotide position 1104 to about 1175 in the Bifidobacterium consensus sequence depicted in Figure 1, and/or capable of specifically binding to a 16S rRNA nucleic acid sequence corresponding to the nucleic acid sequence located from about nucleotide position 1162 to about 1175 in the general consensus sequence depicted in Figure 1, and
- determining whether said probe specifically binds nucleic acid of said sample.

2. A method according to claim 1, further comprising:
- administering to nucleic acid of said sample a probe with a length of between 10 and 50 nucleotides, preferably between 13 and 30 nucleotides, comprising a nucleic acid sequence which is at least 70% homologous to at least part of the sequence TCGCCGCATGTTGC, said part having at least 10 nucleotides, and
- determining whether said probe specifically binds nucleic acid of said sample.

3. A method according to claim 1 or 2, further comprising:
- administering to nucleic acid of said sample a probe with a length of between 10 and 50 nucleotides, preferably between 13 and 30 nucleotides, comprising a nucleic acid sequence which is at least 70% homologous to at least part of the sequence TCGCCCCGTGTTGC, said part having at least 10 nucleotides, and
- determining whether at least one of said probes specifically binds nucleic acid of said sample.

4. A method according to any one of claims 1-3, wherein nucleic acid of said sample is subjected to a nucleic acid amplification reaction.

5. A method according to claim 4, wherein said nucleic acid amplification reaction is performed with a primer with a length of between 9 and 50 nucleotides, preferably with a length of between 9 and 40 nucleotides, more preferably with a length of between 15 and 25 nucleotides, which primer is capable of specifically hybridizing to a 16S rRNA nucleic acid sequence corresponding to the nucleic acid sequence located from about nucleotide position 969 to about 989 in the Bifidobacterium consensus sequence depicted in Figure 1, and/or capable of specifically hybridizing to a 16S rRNA nucleic acid sequence corresponding to the nucleic acid sequence located from about nucleotide position 1023 to about 1043 in the general consensus sequence depicted in Figure 1.

6. A method according to claim 5, wherein said nucleic acid amplification reaction is performed with a primer with a length of between 9 and 50 nucleotides, preferably with a length of between 9 and 40 nucleotides, more preferably with a length of between 15 and 25 nucleotides, comprising a nucleic acid sequence which is at least 70% homologous to at least part of the sequence CCTTACCTGGGCTTGACATGT, said part having at least 10 nucleotides.

7. A method according to any one of claims 4-6, wherein said nucleic acid amplification reaction is performed with a primer with a length of between 9 and 50 nucleotides, preferably with a length of between 9 and 40 nucleotides, more preferably with a length of between 15 and 25 nucleotides, which primer is capable of specifically hybridizing to a 16S rRNA nucleic acid sequence corresponding to the nucleic acid sequence located from about nucleotide position 1187 to about 1207 in the Bifidobacterium consensus sequence depicted in Figure 1, and/or capable of specifically hybridizing to a 16S rRNA nucleic acid sequence corresponding to the nucleic acid sequence located from about nucleotide position 1245 to about 1265 in the general consensus sequence depicted in Figure 1.

8. A method according to any one of claims 4-7, wherein said nucleic acid amplification reaction is performed with a primer with a length of between 9 and 50 nucleotides, preferably with a length of between 9 and 40 nucleotides, more preferably with a length of between 15 and 25 nucleotides, comprising a nucleic acid sequence which is at least 70% homologous to at least part of the sequence GACGTAAGGGGCATGATGATC, said part having at least 10 nucleotides.

9. A method according to any one of claims 1-8, further comprising quantifying nucleic acid of at least one member of Bifidobacteria.

10. A method according to any one of claims 1-9, further comprising identifying nucleic acid of at least one member of Bifidobacteria.

11. A method according to any one of claims 1-10, wherein said nucleic acid is obtained from a faeces sample.

12. A method for detecting and/or isolating nucleic acid of at least one member of Bifidobacteria, comprising administering a probe with a length of between 10 and 50 nucleotides, preferably between 13 and 30 nucleotides, comprising a nucleic acid sequence which is at least 70% homologous to at least part of the sequence TCGCCGCATGTTGC and/or TCGCCCCGTGTTGC, said part having at least 10 nucleotides, to nucleic acid of at least one member of Bifidobacteria.

13. A method for subjecting nucleic acid to a nucleic acid amplification reaction comprising providing said nucleic acid with at least one primer and performing a nucleic acid amplification reaction, wherein said nucleic acid is provided with a primer with a length of between 9 and 50 nucleotides, preferably with a length of between 9 and 40 nucleotides, more preferably with a length of between 15 and 25 nucleotides, which primer is capable of specifically hybridizing to a 16S rRNA nucleic acid sequence corresponding to the nucleic acid sequence located from about nucleotide position 969 to about 989 in the Bifidobacterium consensus sequence depicted in Figure 1, and/or capable of specifically hybridizing to a 16S rRNA nucleic acid sequence corresponding to the nucleic acid sequence located from about nucleotide position 1023 to about 1043 in the general consensus sequence depicted in Figure 1.

14. A method according to claim 13, comprising providing said nucleic acid with a primer with a length of between 9 and 50 nucleotides, preferably with a length of between 9 and 40 nucleotides, more preferably with a length of between 15 and 25 nucleotides, comprising a nucleic acid sequence which is at least 70% homologous to at least part of the sequence CCTTACCTGGGCTTGACATGT, said part having at least 10 nucleotides.

15. A method for subjecting nucleic acid to a nucleic acid amplification reaction comprising providing said nucleic acid with at least one primer and performing a nucleic acid amplification reaction, wherein said nucleic acid is provided with a primer with a length of between 9 and 50 nucleotides, preferably with a length of between 9 and 40 nucleotides, more preferably with a length of between 15 and 25 nucleotides, which primer is capable of specifically hybridizing to a 16S rRNA nucleic acid sequence corresponding to the nucleic acid sequence located from about nucleotide position 1187 to about 1207 in the Bifidobacterium consensus sequence depicted in Figure 1, and/or capable of specifically hybridizing to a 16S rRNA nucleic acid sequence corresponding to the nucleic acid sequence located from about nucleotide position 1245 to about 1265 in the general consensus sequence depicted in Figure 1.

16. A method according to claim 15, comprising providing said nucleic acid with a primer with a length of between 9 and 50 nucleotides, preferably with a length of between 9 and 40 nucleotides, more preferably with a length of between 15 and 25 nucleotides, comprising a nucleic acid sequence which is at least 70% homologous to at least part of the sequence GACGTAAGGGGCATGATGATC, said part having at least 10 nucleotides.

17. A nucleic acid sequence with a length of between 10 and 50 nucleotides, preferably between 13 and 30 nucleotides, comprising a sequence which is at least 70% homologous to at least part of the sequence TCGCCGCATGTTGC, said part having at least 10 nucleotides.

18. A nucleic acid sequence with a length of between 10 and 50 nucleotides, preferably between 13 and 30 nucleotides, comprising a sequence which is at least 70% homologous to at least part of the sequence TCGCCCCGTGTTGC, said part having at least 10 nucleotides.

19. A nucleic acid sequence with a length of between 9 and 50 nucleotides, preferably with a length of between 9 and 40 nucleotides, more preferably with a length of between 15 and 25 nucleotides, comprising a sequence which is at least 70% homologous to at least part of the sequence CCTTACCTGGGCTTGACATGT, said part having at least 10 nucleotides.

20. A nucleic acid sequence with a length of between 9 and 50 nucleotides, preferably with a length of between 9 and 40 nucleotides, more preferably with a length of between 15 and 25 nucleotides, comprising a sequence which is at least 70% homologous to at least part of the sequence GACGTAAGGGGCATGATGATC, said part having at least 10 nucleotides.

21. A primer pair comprising a primer consisting of a nucleic acid sequence according to claim 19 and a primer consisting of a nucleic acid sequence according to claim 20.

22. A kit for detecting Bifidobacterial nucleic acid, comprising a probe with a length of between 10 and 50 nucleotides, preferably between 13 and 30 nucleotides, comprising a nucleic acid sequence which is at least 70% homologous to at least part of the sequence TCGCCGCATGTTGC, said part having at least 10 nucleotides.

23. A kit for detecting Bifidobacterial nucleic acid, comprising a probe with a length of between 10 and 50 nucleotides, preferably between 13 and 30 nucleotides, comprising a nucleic acid sequence which is at least 70% homologous to at least part of the sequence TCGCCCCGTGTTGC, said part having at least 10 nucleotides.

24. A kit for detecting Bifidobacterial nucleic acid, comprising a nucleic acid sequence with a length of between 9 and 50 nucleotides, preferably with a length of between 9 and 40 nucleotides, more preferably with a length of between 15 and 25 nucleotides, comprising a sequence which is at least 70% homologous to at least part of the sequence CCTTACCTGGGCTTGACATGT, said part having at least 10 nucleotides.

25. A kit for detecting Bifidobacterial nucleic acid, comprising a nucleic acid sequence with a length of between 9 and 50 nucleotides, preferably with a length of between 9 and 40 nucleotides, more preferably with a length of between 15 and 25 nucleotides, comprising a sequence which is at least 70% homologous to at least part of the sequence GACGTAAGGGGCATGATGATC, said part having at least 10 nucleotides.

26. A method for determining whether a candidate compound is capable of influencing the amount of Bifidobacteria in a population, comprising:
- providing said population with said candidate compound, and
- determining whether nucleic acid of at least one member of Bifidobacteria is present in a sample of said population, using a method according to any one of claims 1-16.

27. A method according to claim 26, further comprising quantifying nucleic acid of at least one member of Bifidobacteria.

28. A method according to claim 26 or 27, wherein said population comprises a population of the gastrointestinal tract.

29. A method according to any one of claims 26-28, further comprising selecting and/or isolating a candidate compound capable of influencing the amount of Bifidobacteria in said population.

30. A method according to claim 29, further incorporating said selected and/or isolated compound into a food product.
